# FASCICULE DE BREVET EUROPEEN

(11) **EP 0 677 582 B1**
(45) Date de publication et mention de la délivrance du brevet: **23.11.2005**
(21) Numéro de dépôt: 95400592.2
(22) Date de dépôt: 17.03.1995
(51) Int. Cl.: C12N 15/12, C12Q 1/68, C07K 14/74, G01N 33/569, C07K 16/28, A61K 38/17

(54) **Transcrits du gène de CMH de classe I HLA-G et leurs applications**
Gentranscripte von MHC Klasse I HLA-G und deren Verwendungen
Gene transcripts of MHC class I HLA-G and their applications

(30) Priorité: 18.03.1994 FR 9403179
(43) Date de publication de la demande: 18.10.1995
(73) Titulaire: COMMISSARIAT A L'ENERGIE ATOMIQUE ( CEA), 75015 Paris (FR)
(72) Inventeur: Carosella, Edgardo Delfino, F-75016 Paris (FR); Moreau, Philippe, F-91170 Viry-Chatillon (FR); Gluckman, Eliane, F-75005 Paris (FR); Kirszenbaum, Marek, F-91400 Orsay (FR)
(74) Mandataire: Orès, Bernard

(56) Documents cités:
- NUCLEIC ACIDS RESEARCH, vol. 18, no. 8, 25 Avril 1990 OXFORD, GRANDE BRETAGNE, page 2189 H. SHUKLA ET AL. 'The mRNA of a human class I gene HLA G/HLA 6.0 exhibits a restricted pattern of expression.'
- PROCEEDINGS OF THE NATIONAL ACADEMY OF SCIENCES OF THE USA, vol. 89, no. 9, 1 Mai 1992 WASHINGTON DC, TATS UNIS, pages 3947-3951, A. ISHITANI ET AL. 'Alternative splicing of HLA-G transcripts yields proteins with primary structures resembling both class I and class II antigens.'
- PROCEEDINGS OF THE NATIONAL ACADEMY OF SCIENCES OF THE USA, vol. 84, no. 24, 15 Décembre 1987 WASHINGTON DC, TATS UNIS, pages 9145-9149, D. GERAGHTY ET AL. 'A human major histocompatibility complex class I gene that encodes a protein with a shortened cytoplasmic segment.'
- CURRENT OPINION IN IMMUNOLOGY, vol. 5, no. 1, 1993 PHILADELPHIA PA, TATS UNIS, pages 3-7, D. GERAGHTY 'Structure of the HLA class I region and expression of its resident genes.'
- THE FASEB JOURNAL, vol. 4, no. 7, 26 Avril 1990 BETHESDA MD, TATS UNIS, page A2216 D. GERAGHTY ET AL. 'Production of monoclonal antibodies specific for the new class I antigen HLA-G and their use to examine expression in trophoblast cells.'
- THE JOURNAL OF EXPERIMENTAL MEDICINE, vol. 174, no. 3, 1 Septembre 1991 NEW YORK, NY, TATS UNIS, pages 737-740, S. SANDERS ET AL. 'Cell-cell adhesion mediated by CD8 and human histocompatibility leukocyte antigen G, a nonclassical major histocompatibility complex class I molecule on cytotrophoblasts.'
- PROCEEDINGS OF THE NATIONAL ACADEMY OF SCIENCES OF THE USA, vol. 91, no. 10, 10 Mai 1994 WASHINGTON DC, TATS UNIS, pages 4209-4213, M. KIRSZENBAUM ET AL. 'An alternatively spliced form of HLA-G mRNA in human trophoblasts and evidence for the presence of HLA-G transcripts in adult lymphocytes.'
- FOLIA BIOLOGICA, vol. 40, no. 6, 1994 R PUBLIQUE TCH QUE, pages 431-438, P. MOREAU ET AL. 'HLA-G mRNA forms in human trophoblasts and peripheral blood lymphocytes: Potential use in prenatal diagnosis.'

## Description

La présente invention est relative à des transcrits du gène du complexe majeur d'histocompatibilité (CMH) de classe I HLA-G, présents dans les trophoblastes foetaux et/ou dans les cellules mononucléées circulantes de l'adulte ainsi qu'à leurs applications.

Les antigènes du complexe majeur d'histocompatibilité (CMH), se divisent en plusieurs classes, les antigènes de classe I (HLA-A, HLA-B et HLA-C) qui présentent 3 domaines globulaires (α1, α2 et α3), et dont le domaine α3 est associé à la β2 microglobuline, les antigènes de classe II ((HLA-DP, HLA-DQ et HLA-DR) et les antigènes de classe III (complément).

Les antigènes de classe I comprennent, outre les antigènes précités, d'autres antigènes, dits antigènes de classe I non classiques, et notamment les antigènes HLA-E, HLA-F et HLA-G ; ce dernier, en particulier, est exprimé par les trophoblastes extravilleux du placenta humain normal.

La séquence du gène HLA-G (gène HLA-6.0) a été décrite par GERAGHTY et al., (Proc. Natl. Acad. Sci. USA, 1987, 84, 9145-9149) : il comprend 4396 paires de bases et présente une organisation intron/exon homologue à celle des gènes HLA-A, -B et -C. De manière plus précise, ce gène comprend 8 exons et une extrémité non traduite 3'UT, correspondant respectivement : exon 1 : séquence signal, exon 2 : domaine α1, exon 3 : domaine α2, exon 4 : domaine α3, exon 5 : région transmembranaire, exon 6 : domaine cytoplasmique I, exon 7 : domaine cytoplasmique II, exon 8 : domaine cytoplasmique III et région 3' non traduite (GERAGHTY et al., précité, ELLIS et al., J. Immunol., 1990, 144, 731-735). Toutefois le gène HLA-G diffère des autres gènes de classe I, en ce que le codon de terminaison de traduction, en phase, est localisé au niveau du deuxième codon de l'exon 6 ; en conséquence, la région cytoplasmique de la protéine codée par ce gène HLA-6.0 est considérablement plus courte que celle des régions cytoplasmiques des protéines HLA-A, -B et -C.

Contrairement aux autres antigènes de classe I, cet antigène HLA-G (clones G 6.0 et BeWO.G7) ne serait pas polymorphique et ne serait pas exprimé dans d'autres types cellulaires que les trophoblastes (ELLIS et al., J. Immunol., 1990, précité).

D'autres clones HLA-G ont été isolés (TAMAKI et al., Microbiol. Immunol., 1993, 37, 8, 633-640) ; en particulier, le clone HLA-G, dénommé 7.0E, a été isolé d'un placenta japonais et sa séquence en aminoacide s'est révélée identique à celle des clones précités G6.0 et BeWO.G7. Les Auteurs de cet article montrent, en outre, qu'il peut exister une certaine hétérogénéité dans les gènes HLA-G.

Ces antigènes HLA-G sont essentiellement exprimés par les cellules cytotrophoblastiques du placenta ; toutefois, l'ARNm HLA-G a été retrouvé dans les tissus de l'oeil et dans le foie foetal (ISHITANI et al., Proc. Natl. Acad. Sci. USA, 1992, 89, 3947-3951 ; dont la numérotation correspond à celle de la séquence HLA 6.0 telle que décrite dans SHUKLA et al., Nucleic Acids Research, 1990, 18, 8, 2189).

Les antigènes HLA-G exprimés par les cytotrophoblastes sont considérés comme jouant un rôle dans la protection du placenta (absence de rejet). En outre, dans la mesure où l'antigène HLA-G est monomorphique, il peut également être impliqué dans la croissance ou la fonction des cellules placentaires (KOVATS et al., Science, 1990, 248, 220-223).

D'autres recherches concernant cet antigène non classique de classe I (ISHITANI et al., Proc. Natl. Acad. Sci. USA, 1992, 89, 3947-3951) ont montré que le transcrit primaire du gène HLA-G peut être épissé de plusieurs manières et produit au moins 3 ARNm matures distincts : le transcrit primaire d'HLA-G fournit une copie complète (G1) de 1 200 bp, un fragment de 900 bp (G2) et un fragment de 600 bp (G3).

Le transcrit G1 ne comprend pas l'exon 7 et correspond à la séquence décrite par ELLIS et al. (précité), c'est-à-dire qu'il code pour une protéine qui comprend une séquence leader, trois domaines externes, une région transmembranaire et une séquence cytoplasmique. L'ARNm G2 ne comprend pas l'exon 3, c'est-à-dire qu'il code pour une protéine dans laquelle les domaines α1 et α3 sont directement joints ; l'ARNm G3 ne contient ni l'exon 3, ni l'exon 4, c'est-à-dire qu'il code pour une protéine dans laquelle le domaine α1 et la séquence transmembranaire sont directement joints.

L'épissage qui prévaut pour l'obtention de l'antigène HLA-G2 entraîne la jonction d'une adénine (A) (provenant du domaine codant pour α1) avec une séquence AC (issue du domaine codant pour α3), ce qui entraîne la création d'un codon AAC (asparagine) à la place du codon GAC (acide aspartique), rencontré au début de la séquence codant pour le domaine α3 dans HLA-G1.

L'épissage généré pour l'obtention de HLA-G3 n'entraîne pas la formation d'un nouveau codon dans la zone d'épissage.

Les Auteurs de cet article ont également analysés les différentes protéines exprimées : les 3 ARNm sont traduits en protéine dans la lignée cellulaire .221-G.

Les Auteurs de cet article concluent à un rôle fondamental de l'HLA-G dans la protection du placenta vis-à-vis d'une réponse immune maternelle (induction d'une tolérance immune). Toutefois, il est précisé que le rôle de la protéine G3, qui ne contient pas le domaine α3 n'est pas établi.

La complexité du CMH et le rôle de l'antigène HLA-G dans les mécanismes de tolérance ont conduit les Inventeurs à rechercher au moins un transcrit HLA-G, qui pourrait à la fois :
- aisément être mis en évidence dans le sang périphérique,
- serait apte à exprimer, dans des conditions appropriées, une protéine convenable, de préférence soluble, en tant qu'agent de tolérance,
- permettrait de sélectionner des cellules souches immatures aptes à être utilisées dans des greffes de moelle osseuse,
- et permettrait également de mettre en évidence dans le sang maternel, des cellules foetales dans lesquelles ce gène s'exprime.

En conséquence, la présente invention s'est donné pour but de pourvoir à des séquences issues d'un ARNm du gène HLA-G, aptes à résoudre l'ensemble des problèmes exposés ci-dessus.

De telles séquences trouvent application notamment :
- dans la séparation, dans un échantillon de sang maternel, des cellules foetales,
- dans un procédé d'enrichissement en cellules souches immatures, aptes à être utilisées dans les greffes de la moelle et
- dans la séparation spécifique des cellules mononucléées circulantes
- et dans la préparation d'un médicament immunomodulateur.

La présente invention a pour objet une molécule d'ADNc, issue d'un ARNm du gène HLA-G humain du complexe majeur d'histocompatibilité (CMH), caractérisée en ce qu'elle est constituée successivement de 5' en 3', par les fragments suivants dudit gène HLA-G :
- le fragment codant pour le peptide signal (exon 1),
- le fragment codant pour le domaine α1 (exon 2),
- le fragment codant pour le domaine α2 (exon 3),
- le fragment codant pour le domaine transmembranaire TM (exon 5),
- le fragment codant pour le domaine cytoplasmique (exon 6) et
- le fragment 3' non traduit (exon 8), laquelle molécule d'ADNc est dénommée HLA-G 3-5 ou selon les conventions actuellement en vigueur HLA-G4.

Une telle séquence a la particularité de ne pas inclure l'exon 4 et de présenter l'ensemble des propriétés énumérées ci-dessus, et d'être notamment détectable dans les cellules mononucléées circulantes de l'adulte.

On entend par cellules mononucléées, toutes les cellules mononucléées du sang périphérique, à l'exception des cellules tueuses (cellules NK et autres LGL (*large granular lymphocytes*).

La présente invention a également pour objet un fragment de la molécule d'ADNc telle que définie ci-dessus, lequel fragment est constitué successivement de 5' en 3' par :
- le fragment codant pour le domaine α2 (exon 3),
- le fragment codant pour le domaine transmembranaire TM (exon 5),
- le fragment codant pour le domaine cytoplasmique (exon 6) et
- le fragment 3' non traduit (exon 8).

Conformément à l'invention, une telle séquence qui code pour une protéine dans laquelle le domaine α2 et la séquence transmembranaire HLA-G sont directement joints, présente la SEQ ID NO: 1 suivante : dans laquelle
- X₁: représente G ou A,
- X₂: représente C ou G,
- X₃: représente T ou C,
et comprend 0,43 kb.

De manière inattendue, un tel transcrit est détecté aussi bien dans les trophoblastes (premier trimestre de gestation) que dans les cellules mononucléées circulantes chez l'adulte.

La présente invention a également pour objet un produit de transcription du gène HLA-G humain du CMH, isolé et purifié, caractérisé en ce qu'il est constitué, successivement de 5' en 3', par les fragments suivants du gène HLA-G :
- le fragment codant pour le peptide signal (exon 1),
- le fragment codant pour le domaine α1 (exon 2),
- le fragment codant pour le domaine α2 (exon 3),
- le fragment codant pour le domaine transmembranaire TM (exon 5), et
- le fragment codant pour le domaine cytoplasmique (exon 6) de HLA-G.

La présente invention a également pour objet des fragments oligonucléotidiques de la molécule d'ADNc, ne comprenant pas l'exon 4, conforme à l'invention ; parmi ces fragments, dont la numérotation correspond à celle de la séquence HLA 6.0, telle que décrite dans SHUKLA et al. précité, on peut citer :
- GGA AGA GGA GAC ACCG GAA CA (SEQ ID NO: 2), dénommé G.257 (+), situé au niveau de l'exon 2 et correspondant au fragment 257-276 de ladite séquence d'ADNc ;
- CCA ATG TGG CTG AAC AAA GG (SEQ ID NO: 3), dénommé G.526 (+), situé au niveau de l'exon 3 et correspondant au fragment 526-545 de ladite séquence d'ADNc ;
- CCC CTT TTC TGG AAC AGG AA (SEQ ID NO: 4), dénommé G. 1200 (-) et correspondant au fragment 1200-1219 de ladite séquence d'ADNc ;
- TGA GAC AGA GAC GGA GAC AT (SEQ ID NO: 5), dénommé G.1225 (-) et correspondant au fragment 1225-1244 de ladite séquence d'ADNc ;
- CAG CGC GCG GAG CAG TCT TC (SEQ ID NO: 6), dénommé G.3.5 (+) et correspondant à la jonction exon 3-exon 5 de ladite séquence d'ADNc.

La présente invention a également pour objet des sondes nucléotidiques, caractérisées en ce qu'elles sont constituées par les molécules d'acide nucléique et les fragments de celles-ci tels que définis ci-dessus, marqués à l'aide d'un marqueur tel qu'un isotope radioactif, une enzyme appropriée ou un fluorochrome.

Selon un mode de réalisation avantageux de ladite sonde, elle présente la séquence ID NO: 6 ci-dessus, spécifique du transcrit, sans exon 4, conforme à l'invention.

Selon un autre mode de réalisation avantageux de ladite sonde, elle présente la séquence ID NO: 4 ci-dessus ; une telle sonde est apte à détecter tous les produits de transcription du gène HLA-G humain du CMH.

La présente invention a également pour objet des amorces, aptes à être utilisées pour amplifier une séquence nucléotidique conforme à l'invention, telle que définie ci-dessus (séquence sans exon 4).

Selon un mode de réalisation avantageux desdites amorces, une paire d'amorces préférée comprend :
(1)
(2)

Selon un autre mode de réalisation avantageux desdites amorces, une autre paire d'amorces préférée comprend :
(3)
(4)

La présente invention a également pour objet des protéines ou fragments peptidiques, caractérisés en ce qu'ils sont codés par les molécules d'acide nucléique et les fragments de ceux-ci, tels que définis ci-dessus.

Selon un mode de réalisation avantageux de l'invention, ledit peptide est codé par un fragment du transcrit HLA-G4 et répond à la SEQ ID NO: 7 ci-après :

Selon un autre mode de réalisation avantageux des peptides conformes à l'invention, ils peuvent être obtenus par synthèse.

Conformément à l'invention, de tels protéines ou peptides trouvent application en tant que médicaments, notamment dans des compositions immunomodulatrices.

La présente invention a également pour objet un procédé de sélection et d'enrichissement en cellules hématopoïétiques indifférenciées (cellules sanguines immatures ou cellules souches), caractérisé en ce qu'il comprend :
(a) le prélèvement d'un échantillon sélectionné, selon le cas, parmi le sang périphérique, le sang de cordon ombilical ou la moelle osseuse,
(b) la mise en contact dudit échantillon avec des anticorps anti-CD34,
(c) la séparation des complexes cellules contenant un antigène CD34-anticorps anti-CD34 formés,
(d) la réalisation d'une RT-PCR *in situ* sur les cellules obtenues à l'étape (c) en présence d'une paire d'amorces marquées à l'aide d'un fluorochrome, conforme à l'invention,
(e) la séparation des cellules fluorescentes, et
(f) la sélection des cellules non fluorescentes immatures pluripotentes.

Les cellules obtenues en (f) sont des cellules CD34⁺ HLA-G⁻, qui sont les cellules les plus immatures. Un tel procédé permet avantageusement d'obtenir un grand nombre de cellules souches immatures aptes à être utilisées pour des greffes de cellules souches.

Selon un mode de mise oeuvre avantageux dudit procédé, la paire d'amorces est sélectionnée parmi les paires (1)-(2) et (3)-(4) telles que définies ci-dessus.

La présente invention a également pour objet un procédé de détection et de séparation de cellules exprimant le transcrit HLA-G4, conforme à l'invention, caractérisé en ce qu'il comprend, *in situ*, la mise en oeuvre d'une RT-PCR, par :
(a) mise en contact de cellules sanguines avec une paire d'amorces marquées, conforme à l'invention, sélectionnée parmi les paires (1)-(2) et (3)-(4) telles que définies ci-dessus, et
(b) séparation des cellules marquées par tout moyen convenable, notamment par cytofluorométrie.

La RT-PCR est notamment décrite dans American J. Pathol., 1993, 143, 6, 1527-1534.

La présente invention a également pour objet des anticorps, caractérisés en ce qu'ils sont préparés par immunisation d'un animal approprié avec une protéine ou un peptide selon l'invention, et en ce qu'ils reconnaissent parmi les protéines HLA-G, uniquement ladite protéine ou ledit peptide selon l'invention.

La présente invention a également pour objet un procédé de séparation de cellules foetales nucléées, à partir d'un échantillon de sang maternel, caractérisé en ce qu'il comprend :
(1) la mise en contact de l'échantillon de sang maternel avec des anticorps dirigés contre les protéines HLA-G telles que définies ci-dessus, et
(2) la séparation des complexes cellules foetales-anticorps obtenus.

Un tel procédé permet le dépistage prénatal d'anomalies foetales et notamment d'aberrations chromosomiques ou d'aberrations géniques et un dépistage prénatal du sexe, à partir des cellules foetales circulantes ainsi isolées, de manière fiable, de la circulation sanguine maternelle, dans la mesure où seules ces cellules foetales sont porteuses desdites protéines HLA-G.

La présente invention a également pour objet un procédé de séparation spécifique de lymphocytes circulants, caractérisé en ce qu'il comprend, la mise en oeuvre d'une RT-PCR *in situ*, par :
(a) mise en contact de cellules sanguines avec une paire d'amorces marquées conforme à l'invention et
(b) séparation des cellules marquées (cytofluorométrie...).

Un tel procédé permet, si nécessaire, de séparer en outre les cellules mononucléées n'exprimant pas le gène HLA-G (cellules tueuses, notamment) des autres cellules mononucléées.

En outre, seules les cellules NK (ou cellules tueuses) n'expriment pas l'ARNm d'HLA-G ; ceci montre que les produits d'expression du gène HLA-G protègent contre l'activité lytique des cellules NK.Outre les dispositions qui précèdent, l'invention comprend encore d'autres dispositions, qui ressortiront de la description qui va suivre, qui se réfère à des exemples de mise en oeuvre du procédé objet de la présente invention.

Il doit être bien entendu, toutefois, que ces exemples sont donnés uniquement à titre d'illustration de l'objet de l'invention, dont ils ne constituent en aucune manière une limitation.

### EXEMPLE 1 : Transcrit du gène HLA-G épissé, sans exon 4.

### a) Obtention des cellules adultes et des tissus foetaux :

- Les trophoblastes du premier trimestre de gestation sont obtenus lors d'IVG (6-10 semaines de gestation).
- Les foies foetaux du deuxième trimestre sont obtenus, lors d'interruptions thérapeutiques de grossesse (16 semaines de gestation).
   Les tissus sont lavés dans un tampon PBS et les trophoblastes ou le foie sont identifiés au microscope et congelés dans de l'azote liquide.
- Des échantillons de sang périphérique humain sont obtenus chez des volontaires (homme normal).

Les cellules mononucléées sont séparés des polynucléaires par centrifugation de densité (Ficoll-Hypaque®) et l'ARNm est isolé des deux populations.

On obtient également des cellules mononucléées enrichies en lymphocytes B par immunoabsorption sur billes magnétiques recouvertes d'anticorps anti-CD 19 (Dynabeads-Dynal/Biosys, France) et des cellules mononucléées enrichies en lymphocytes T, par séparation sur Leuko-Pac® (Fenwal Laboratories, USA).

L'enrichissement est d'environ 90 % pour les cellules B et d'environ 87 % pour les cellules T, après estimation par analyse FACS : évaluation des complexes formés respectivement avec des anticorps anti-CD20 ou des anticorps anti-CD3, marqués au FITC et 1.10⁵ cellules de chaque sous-population.

### b) Isolement de l'ARN et amplification par RT-PCR :

L'ARNm total est isolé à partir d' 1 g de tissu congelé ou de 2.10⁷ cellules, en utilisant le réactif RNA-Zol B® (Bioprobe Systems, France), conformément aux recommandations du fabriquant ; la qualité du produit obtenu est vérifiée par électrophorèse sur gel d'agarose dénaturant à 1,5 %.

L'ADNc est préparé à partir de 10 µg d'ARN total, en présence d'amorce oligo-dT et de transcriptase réverse M-MLV (Gibco-BRL, Life Technologies), par incubation de 20 µl du mélange à 42°C pendant 1 heure, puis à 95°C pendant 5 minutes.

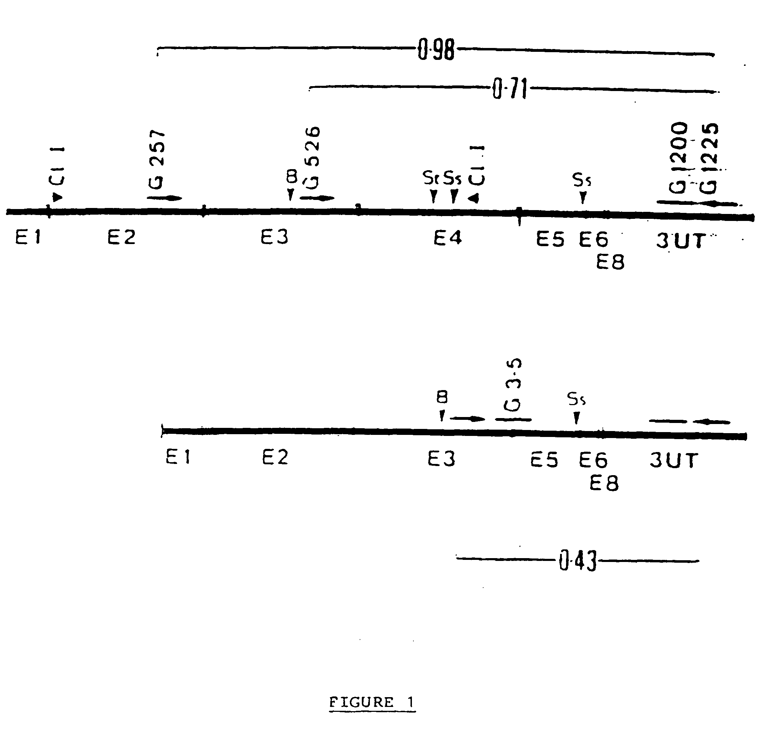

Les fragments PCR qui peuvent être obtenus selon les amorces utilisées (voir Tableau I ci-après), sont représentés à la figure 1.

Pour amplifier le transcrit G.3-5 (maintenant dénommé HLA-G4) conforme à l'invention (fragment de 0,43 kb), on utilise de préférence les amorces G.526 et G.1225 (représentées par des flèches horizontales) ; la sonde utilisée pour détecter ce transcrit amplifié est la sonde G.3-5 (représentée par une ligne épaisse).

Les flèches verticales indiquent les sites de restriction spécifiques des exons 3, 4 et 5, utiles pour l'analyse de restriction des produits de la RT-PCR, clonés dans le vecteur pPCRII (B : BglI ; St : StuI ; Ss : SstI).

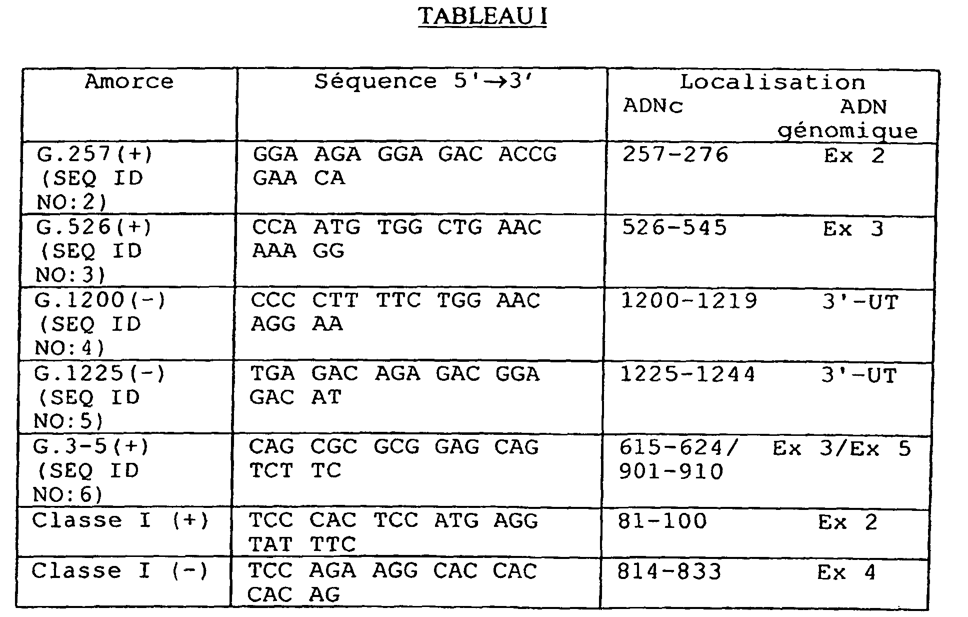

dans lequel Ex. est l'abréviation de exon.

Afin de réduire la quantité de produit amplifié non spécifique, l'amplification est réalisée en utilisant la technique *hot-start* : dans un tube de réaction, 200 µM de chaque dNTP, 0,1 µg de chaque amorce et une pastille d'AmpliWax® (Cetus-Perkin Elmer, France), dans 50 µl d'un tampon PCR 1X, sont incubés à 75°C pendant 5 min ; dans un second tube, 2 µl de la solution de RT ou 1 µg d'ADN génomique et 3,5 U de Taq polymérase (Cetus-Perkin Elmer) dans 50 µl de tampon PCR IX sont incubés à 95°C pendant 5 min.

Le contenu des 2 tubes est ensuite mélangé et soumis à 35 cycles de PCR dans les conditions suivantes :
94°C pendant 1 minute,
61°C pendant 1 minute,
72°C pendant 1 minute 30.

La dernière étape d'élongation à 72°C est de 10 min.

Les produits PCR sont analysés par électrophorèse en gel d'agarose à 1 % et colorés au bromure d'éthidium.

La spécificité des produits obtenus est confirmée par blotting alcalin des fragments (NaOH 0,4 N) sur une membrane de nylon (Hybond N⁺, Amersham, France), puis une hybridation est ensuite réalisée dans un tampon : 5X SSPE, 5X Denhardt, SDS 0,5 %, ADN de sperme de saumon 100 µg/ml, pendant 2 h à 55°C, en présence d'une sonde oligonucléotidique G-1200 marquée au ³²P ([γ-³²P]dATP) et d'un kit de marquage de l'extrémité 5' (Boehringer-Mannheim, France).

Différents contrôles d'amplification sont réalisés : mélange réactionnel RT sans transcriptase réverse M-MLV (RT⁻) et mélange PCR sans matrice d'ADNc (blanc).

Par ailleurs, des contrôles positifs sont réalisés avec des amorces ubiquitaires de la classe I des HLA (voir Tableau I).

### c) Résultats :

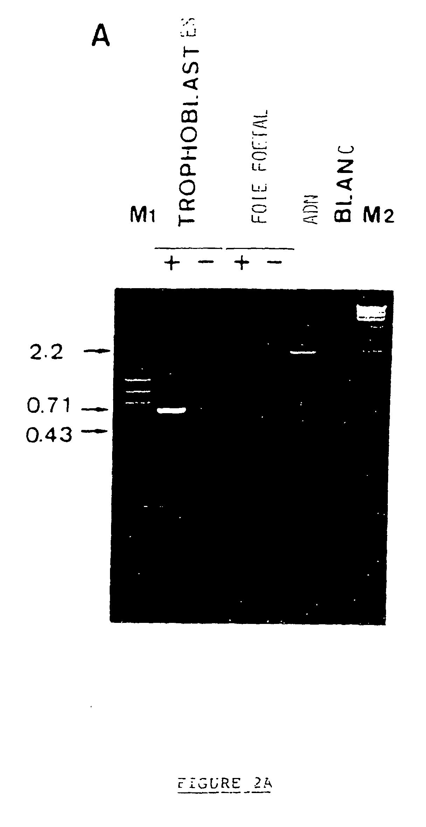

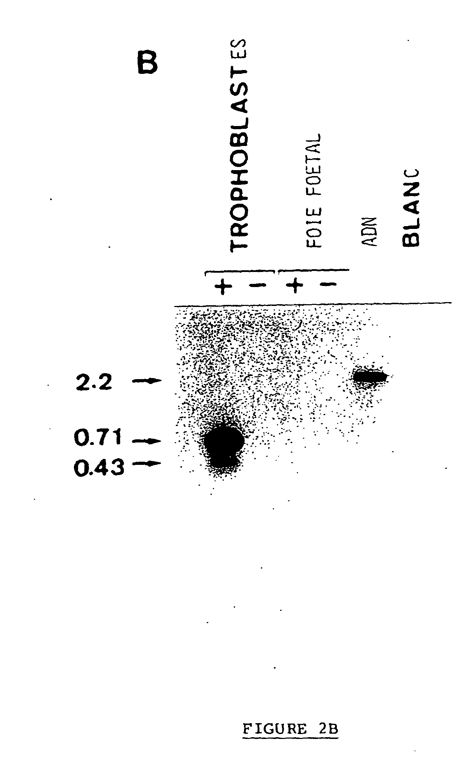

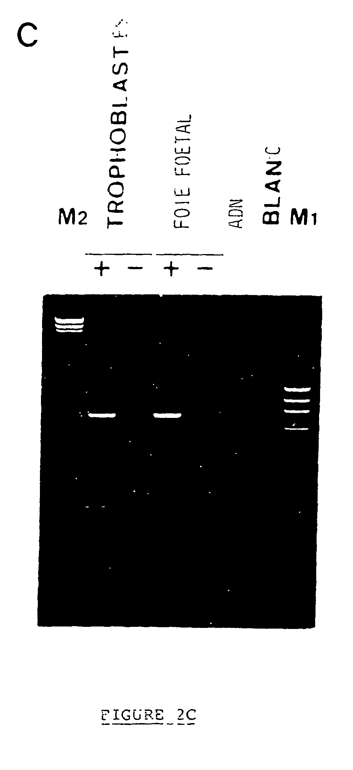

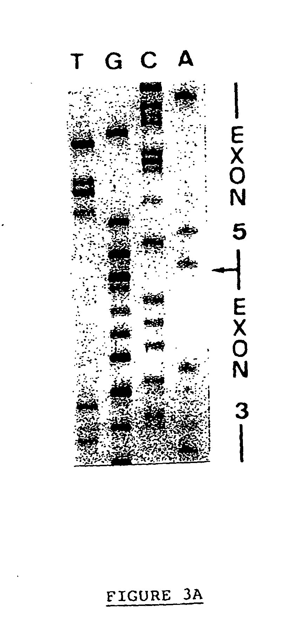

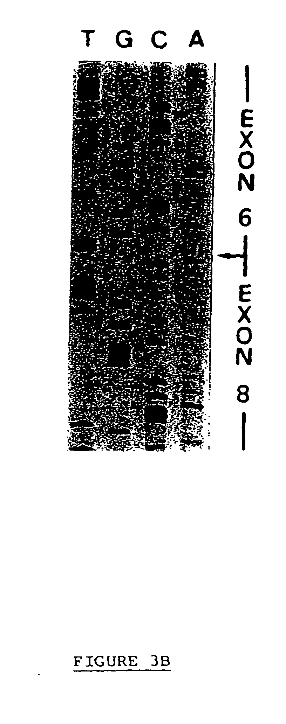

1. Avec les amorces G.526 et G.1225, deux fragments (0,71 kb et 0,43 kb) sont observés après électrophorèse sur gel et coloration au bromure d'éthidium qui s'hybride avec la sonde G 1200 (voir figure 1 et figures 2A et B).
2. La RT-PCR de l'ARNm du foie fétal du 2ème trimestre ne montre aucune bande sur le gel, ni aucun signal d'hybridation, alors que l'amplification avec des amorces des HLA de classe I entraîne un signal positif (figure 2C).
   L'amplification de l'ADN génomique présente une bande à environ 2,2 kb conformément à la séquence génomique d'HLA-G.
3. Le fragment de 0,71 kb correspond au transcrit HLA-G complet tandis que
4. le fragment de 0,43 kb correspond à un transcrit ne contenant pas l'exon 4 (→ 276 bp) (HLA-G4).
   Pour confirmer l'absence de l'exon 4, la bande de 0,43 kb est découpée et séquencée selon la méthode suivante :
   Pour générer des matrices de séquençage, les fragments PCR sont séparés par électrophorèse sur gel de polyacrylamide à 4 %, coupés et élués avec un tampon acétate d'ammonium 0,5 M et EDTA 5 mM et réamplifiés par PCR asymétrique, de manière à générer des produits simple-brin.
   Le produit réamplifié est purifié par extraction au phénol-chloroforme, précipité 2 fois avec de l'éthanol, puis séquencé en utilisant le kit de séquençage T7 Sequenase 2.0 (USB/Touzard-Matignon, France).
   De plus, les produits de la PCR sont clonés dans le vecteur pPCRII, en utilisant le kit TA *Cloning System* (Invitrogen, USA), puis séquencés.
   Comme le montre la figure 3, le fragment de 0,43 kb présente clairement une jonction entre l'exon 3 et l'exon 5, qui résulte de la perte de l'exon 4.
   De plus, le séquençage montre que le transcrit conforme à l'invention ne comprend pas l'exon 7 ; la présence d'un codon stop dans l'exon 6 est en outre observée.
5. Evaluation de la fréquence du transcrit HLA-G4 :
   comme suggéré par la figure 2, l'intensité d'hybridation de la bande de 0,43 kb, correspondant au transcrit épissé, est plus faible que celle correspondant à la copie complète ; ceci suggère que ce transcrit est moins abondant dans la population ARNm d'HLA-G.

De manière à évaluer les quantités relatives de ces 2 transcrits, les produits de l'amplification PCR de l'ARNm des trophoblastes du 1er trimestre avec les amorces G.526-G.1225 sont clonés dans le vecteur pPCRII, comme précisé ci-dessus.

Sur 260 clones analysés par hybridation des réplicats, soit avec la sonde G1200, soit avec la sonde G.3-5, environ 210 clones présentent une hybridation positive avec la sonde G.1200 et seulement un clone est positif avec la sonde G.3-5.

Ce clone unique a été séquencé, tandis que 5 clones positifs avec la sonde G.1200, choisis au hasard, sont analysés par leur carte de restriction vis-à-vis d'enzymes spécifiques des exons 3 et 4 (voir figure 1).

Une comparaison des séquences montre que le clone positif avec la sonde G.3-5 ne comprend pas l'exon 4, tandis que les 5 autres clones correspondent au transcrit complet.

Ainsi, la fréquence du transcrit conforme à l'invention par rapport au transcrit comprenant la séquence complète peut être estimée à environ 1/200.

La sélection de l'amorce G.526 (spécifique de l'exon 3) a permis d'obtenir, lors de l'amplification PCR, la sélection d'un transcrit dépourvu d'exon 4.

L'absence d'exon 4 crée, à la jonction d'épissage un codon GAG (Glu) à la place du codon GAC (Asp) que l'on retrouve dans le transcrit complet.

L'absence d'exon 4 exclut le domaine α3 de la protéine déduite correspondante et confère une nouvelle structure à l'antigène HLA-G, qui peut être exprimé à la surface des cellules trophoblastiques.

Dans cette structure, le domaine α2 et la région transmembranaire sont reliés et peuvent induire des modifications conformationnelles de la protéine de surface.

En particulier, ladite protéine peut présenter une capacité différente de se lier aux peptides.

### EXEMPLE 2 : Expression du transcrit HLA-G complet ou du transcrit sans exon 4 dans les cellules mononucléées circulantes périphériques de l'adulte.

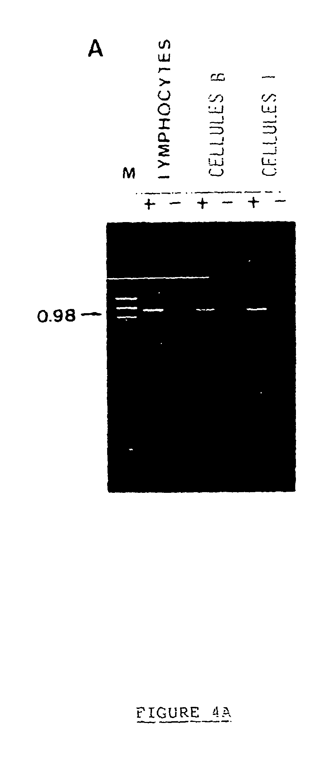

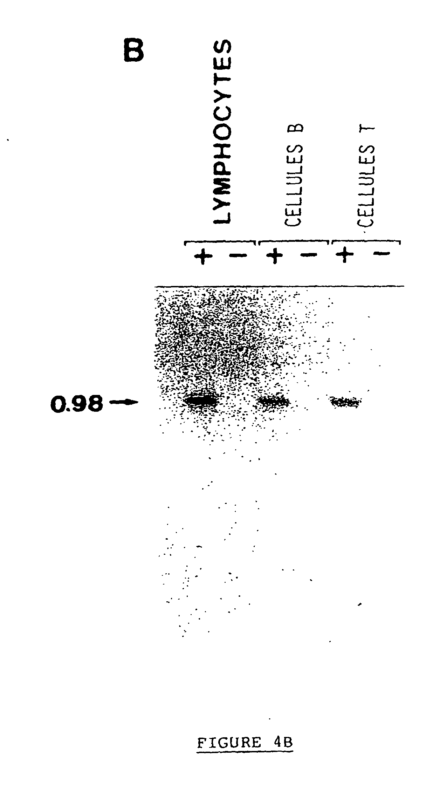

La figure 4 montre les résultats de l'amplification PCR obtenue avec les amorces G.257-G.1225, à partir de matrices d'ADNc de cellules mononucléaires périphériques obtenues chez l'homme (sujets mâles).

Une bande de 1 kb est observée en gel d'agarose (Figure 4A) ; une hybridation avec la sonde G.1200 révèle une bande de même dimension (figure 4B).

Conformément à la séquence d'ADNc, cette bande correspond au transcrit HLA-G complet.

Pour confirmer la production de ce transcrit, le produit de la PCR est cloné dans un vecteur pPCRII puis séquencé, selon la méthode exposée ci-dessus.

Le fragment de 1 kb est entièrement homologue avec la séquence HLA-G décrite par SHUKLA et al. (Nucleic Acids Research, 1990, 18, 8, 2189).

L'amplification PCR de l'ADNc à partir d'une population de polynucléaires avec des amorces spécifiques HLA-G génère une bande de faible intensité de même dimension, 0,71 kb, que celle observée avec les cellules mononucléées (contamination de la fraction polynucléaire par des cellules mononucléées).

Pour préciser la spécificité cellulaire de l'expression du gène HLA-G chez les lymphocytes circulants de l'adulte, les populations mononucléaires ont été séparées et une PCR a été réalisée avec des amorces spécifiques HLA-G sur l'ADNc obtenu à partir de sous-populations enrichies en cellules T ou en cellules B.

Une bande de 1 kb est observée pour les fractions cellules T aussi bien en gel d'agarose qu'en analyse par blot après hybridation avec la sonde G.1200 (figures 4A et B) (transcrit complet).

L'utilisation d'une technique PCR *hot-start* a permis de mettre en évidence la présence d'ARNm de HLA-G dans les lymphocytes périphériques de l'adulte, ce transcrit étant présent à la fois dans les cellules B et les cellules T.

Tous les transcrits alternatifs sont observés pour les cellules mononucléées du sang périphérique.

## Revendications

1. Molécule d'ADNc, issue d'un ARNm du gène HLA-G humain du complexe majeur d'histocompatibilité (CMH), **caractérisée en ce qu'**elle est constituée, successivement de 5' en 3', par les fragmentes suivants dudit gène HLA-G :
- le fragment codant pour le peptide signal (exon 1),
- le fragment codant pour le domaine α₁ (exon 2),
- le fragment codant pour le domaine α₂ (exon 3),
- le fragment codant pour le domaine transmembranaire TM (exon 5),
- le fragment codant pour le domaine cytoplasmique (exon 6) et
- le fragment 3' non traduit (exon 8), laquelle molécule d'ADNc est dénommée HLA-G4.

2. Fragment de la molécule d'ADNc selon la revendication 1, **caractérisé en ce qu'**il est constitué successivement de 5' en 3' par :
- le fragment codant pour le domaine α2 (exon 3),
- le fragment codant pour le domaine transmembranaire TM (exon 5),
- le fragment codant pour le domaine cytoplasmique (exon 6) et
- le fragment 3' non traduit (exon 8).

3. Fragment selon la revendication 2, **caractérisé en ce qu'**il est défini par la séquence SEQ ID NO: 1, codant pour une protéine dans laquelle le domaine α₂ et la séquence transmembranaire d'HLA-G sont directement joints.

4. Produit de transcription du gène HLA-G humain du CMH, isolé et purifié, **caractérisé en ce qu'**il est constitué, successivement de 5' en 3', par les fragments suivants du gène HLA-G :
- le fragment codant pour le peptide signal (exon 1),
- le fragment codant pour le domaine α1 (exon 2),
- le fragment codant pour le domaine α2 (exon 3),
- le fragment codant pour le domaine transmembranaire TM (exon 5), et
- le fragment codant pour le domaine cytoplasmique (exon 6) de HLA-G.

5. Fragment de la molécule d'ADNc selon la revendication 1, **caractérisé en ce qu'**il est défini par l'oligonucléotide de séquence SEQ ID NO:2.

6. Fragment de la molécule d'ADNc selon la revendication 1, **caractérisé en ce qu'**il est défini par l'oligonucléotide de séquence SEQ ID NO:3.

7. Fragment de la molécule d'ADNc selon la revendication 1, **caractérisé en ce qu'**il est défini par l'oligonucléotide de séquence SEQ ID NO: 4.

8. Fragment de la molécule d'ADNc selon la revendication 1, **caractérisé en ce qu'**il est défini par l'oligonucléotide de séquence SEQ ID NO: 5.

9. Fragment de la molécule d'ADNc selon la revendication 1, **caractérisé en ce qu'**il est défini par l'oligonucléotide de séquence SEQ ID NO: 6:

10. Sondes nucléotidiques, **caractérisées en ce qu'**elles sont sélectionnées dans le groupe constitué par les molécules ou les fragments d'acide nucléique selon l'une quelconque de revendications 1 à 9, marqués à l'aide d'un marqueur tel qu'un isotope radioactif, une enzyme appropriée ou un fluorochrome.

11. Sonde selon la revendication 10, **caractérisée en ce qu'**elle est constituée par un oligonucléotide de séquence SEQ ID NO: 6 selon la revendication 9.

12. Sonde selon la revendication 10, **caractérisée en ce qu'**elle est constituée par un oligonucléotide de séquence SEQ ID NO: 4 selon la revendication 7.

13. Paires d'amorces pour l'amplification d'une séquence selon l'une quelconque des revendications 1 à 4, **caractérisées en ce qu'**elles sont sélectionnées dans le groupe constitué par les oligonucléotides de séquence SEQ ID NO:2 à 6.

14. Paire d'amorces selon la revendication 13, **caractérisée en ce qu'**elle est constituée par l'oligonucléotide de séquence SEQ ID NO: 2 selon la revendication 5, apparié à l'oligonucléotide de séquence SEQ ID NO: 5 selon la revendication 8.

15. Paire d'amorces selon la revendication 13, **caractérisée en ce qu'**elle est constituée par l'oligonucléotide de séquence SEQ ID NO: 3 selon la revendication 6, apparié à l'oligonucléotide de séquence SEQ ID NO: 5 selon la revendication 8.

16. Protéine ou fragment peptidique, **caractérisé en ce que** sa séquence est celle qui est codée par la molécule ou le fragment d'acide nucléique selon l'une quelconque des revendications 1 à 4.

17. Peptide selon la revendication 16, **caractérisé en ce qu'**il est défini par la séquence SEQ ID NO: 7.

18. Procédé de sélection et d'enrichissement en cellules hématopoïétiques indifférenciées (cellules sanguines immatures ou cellules souches), **caractérisé en ce qu'**il comprend :
(a) la sélection, selon le cas, parmi le sang périphérique, le sang de cordon ombilical ou la moelle osseuse, d'un échantillon ayant été prélevé,
(b) la mise en contact dudit échantillon avec des anticorps anti-CD34,
(c) la séparation des complexes cellules contenant un antigène CD34-anticorps anti-CD34 formés,
(d) la réalisation d'une RT-PCR *in situ* sur les cellules obtenues à l'étape (c) en présence d'amorces marquées à l'aide d'un fluorochrome, telles que définies à l'une quelconque des revendications 13 à 15,
(e) la séparation des cellules fluorescentes, obtenues et
(f) la sélection des cellules non fluorescentes immatures pluripotentes.

19. Procédé de détection et de séparation de cellules exprimant le transcrit selon l'une quelconque des revendications 1 à 4, **caractérisé en ce qu'**il comprend, *in situ*, la mise en oeuvre d'une RT-PCR, par :
(a) mise en contact de cellules sanguines avec une paire d'amorces marquées telles que définies à l'une quelconque des revendications 13 à 15, et
(b) séparation des cellules marquées par tout moyen convenable.

20. Anticorps, **caractérisés en ce qu'**ils sont préparés par immunisation d'un animal approprié, avec une protéine selon la revendication 16 ou un peptide selon la revendication 17 et **en ce qu'**ils reconnaissent parmi les protéines HLA-G, uniquement ladite protéine selon la revendication 16 ou ledit peptide selon la revendication 17.

21. °) Procédé de séparation de cellules foetales nucléées, à partir d'un échantillon de sang maternel, **caractérisé en ce qu'**il comprend :
(1) la mise en contact de l'échantillon de sang maternel avec des anticorps dirigés contre les protéines HLA-G selon la revendication 16, et
(2) la séparation des complexes cellules foetales-anticorps obtenus.

22. Procédé de séparation spécifique de lymphocytes circulants, **caractérisé en ce qu'**il comprend, *in situ*, la mise en oeuvre d'une RT-PCR, par :
(a) mise en contact de cellules sanguines avec une paire d'amorces marquées telles que définies à l'une quelconque des revendications 13 à 15 et
(b) séparation convenable des cellules marquées.

23. Médicament, **caractérisé en ce qu'**il comprend une protéine selon la revendication 16 ou un peptide selon la revendication 17.

24. Compositions immunotolérantes, **caractérisées en ce qu'**elles comprennent une protéine selon la revendication 16 ou un peptide selon la revendication 17, associé à un véhicule ou un support convenable du point de vue pharmaceutique.

## Patentansprüche

1. cDNA-Molekül, das von einer mRNA des humanen HLA-G-Gens des Haupthistokompatibilitätskomplexes (MHC) abgeleitet ist, **dadurch gekennzeichnet, dass** es aufeinander folgend von 5' nach 3' aus den folgenden Fragmenten des HLA-G-Gens besteht:
- dem Fragment, das für das Peptidsignal codiert, (Exon 1),
- dem Fragment, das für die α₁-Domäne codiert, (Exon 2),
- dem Fragment, das für die α₂-Domäne codiert, (Exon 3),
- dem Fragment, das für die Transmembrandomäne TM codiert (Exon 5),
- dem Fragement, das für die cytoplasmische Domäne codiert, (Exon 6), und
- dem nicht-translatierten 3'-Fragment, (Exon 8), wobei das cDNA-Molekül HLA-G4 bezeichnet wird.

2. Fragment des cDNA-Moleküls nach Anspruch 1, **dadurch gekennzeichnet, dass** es aufeinander folgend von 5' nach 3' aus:
- dem Fragment, das für die α₂-Domäne codiert, (Exon 3),
- dem Fragment, das für die Transmembrandomäne TM codiert, (Exon 5),
- dem Fragment, das für die cytoplasmische Domäne codiert, (Exon 6), und
- dem nicht-translatierten 3'-Fragment, (Exon 8), besteht.

3. Fragment nach Anspruch 2, **dadurch gekennzeichnet, dass** es durch die Sequenz SEQ ID NO:1 definiert ist, die für ein Protein codiert, in dem die α₂-Domäne und die Transmembransequenz von HLA-G direkt verbunden sind.

4. Transkriptionsprodukt des humanen HLA-G-Gens des MHC, das isoliert und gereinigt ist, **dadurch gekennzeichnet, dass** es aufeinander folgend von 5' nach 3' aus den folgenden Fragmenten des HLA-G-Gens besteht:
- dem Fragment, das für das Peptidsignal codiert, (Exon 1),
- dem Fragment, das für die α₁-Domäne codiert, (Exon 2),
- dem Fragment, das für die α₂-Domäne codiert, (Exon 3),
- dem Fragment, das für die Transmembrandomäne TM codiert, (Exon 5), und
- dem Fragment, das für die cytoplasmische Domäne von HLA-G codiert, (Exon 6).

5. Fragment des cDNA-Moleküls nach Anspruch 1, **dadurch gekennzeichnet, dass** es durch das Oligonukleotid der Sequenz SEQ ID NO:2 definiert ist.

6. Fragment des cDNA-Moleküls nach Anspruch 1, **dadurch gekennzeichnet, dass** es durch das Oligonukleotid der Sequenz SEQ ID NO:3 definiert ist.

7. Fragment des cDNA-Moleküls nach Anspruch 1, **dadurch gekennzeichnet, dass** es durch das Oligonukleotid der Sequenz SEQ ID NO:4 definiert ist.

8. Fragment des cDNA-Moleküls nach Anspruch 1, **dadurch gekennzeichnet, dass** es durch das Oligonukleotid der Sequenz SEQ ID NO:5 definiert ist.

9. Fragment des cDNA-Moleküls nach Anspruch 1, **dadurch gekennzeichnet, dass** es durch das Oligonukleotid der Sequenz SEQ ID NO:6 definiert ist.

10. Nukleotidsonden, **dadurch gekennzeichnet, dass** sie aus der Gruppe, bestehend aus den Nukleinsäuremolekülen oder Nukleinsäurefragmenten nach einem der Ansprüche 1 bis 9, die mit Hilfe eines Markers wie einem radioaktiven Isotop, einem geeigneten Enzym oder einem Fluorochrom markiert sind, ausgewählt sind.

11. Sonde nach Anspruch 10, **dadurch gekennzeichnet, dass** sie durch ein Oligonukleotid der Sequenz SEQ ID NO:6 nach Anspruch 9 gebildet wird.

12. Sonde nach Anspruch 10, **dadurch gekennzeichnet, dass** sie durch ein Oligonukleotid der Sequenz SEQ ID NO:4 nach Anspruch 7 gebildet wird.

13. Primerpaare zur Amplifikation einer Sequenz nach einem der Ansprüche 1 bis 4, **dadurch gekennzeichnet, dass** sie aus der Gruppe, bestehend aus den Oligonukleotiden der Sequenz SEQ ID NO:2 bis 6 ausgewählt sind.

14. Primerpaar nach Anspruch 13, **dadurch gekennzeichnet, dass** es durch das Oligonukleotid der Sequenz SEQ ID NO:2 nach Anspruch 5, gepaart mit dem Oligonukleotid der Sequenz SEQ ID NO:5 nach Anspruch 8 gebildet wird.

15. Primerpaar nach Anspruch 13, **dadurch gekennzeichnet, dass** es durch das Oligonukleotid der Sequenz SEQ ID NO:3 nach Anspruch 6, gepaart mit dem Oligonukleotid der Sequenz SEQ ID NO:5 nach Anspruch 8 gebildet wird.

16. Protein oder Peptidfragment, **dadurch gekennzeichnet, dass** seine Sequenz die ist, die durch das Nukleinsäuremolekül oder das Nukleinsäurefragment nach einem der Ansprüche 1 bis 4 codiert wird.

17. Peptid nach Anspruch 16, **dadurch gekennzeichnet, dass** es durch die Sequenz SEQ ID NO:7 definiert ist.

18. Verfahren zur Selektion und Anreicherung von undifferenzierten hämatopoetischen Zellen (unreife Blutzellen oder Stammzellen), **dadurch gekennzeichnet, dass** es umfasst:
(a) gegebenenfalls Selektion einer Probe, die vorher entnommen worden war aus peripherem Blut, Nabelschnurblut oder Knochenmark;
(b) Inkontaktbringen der Probe mit Antikörpern gegen CD34;
(c) Abtrennung der Komplex Antigen-CD34 enthaltende Zellen-Anti-CD34-Antikörper;
(d) Durchführung einer RT-PCR in situ mit den in Stufe (c) erhaltenen Zellen in Gegenwart von mit Hilfe eines Fluorochroms markierten Primern, wie sie in einem der Ansprüche 13 bis 15 definiert sind;
(e) Abtrennung der erhaltenen fluoreszierenden Zellen und
(f) Selektion der nicht-fluoreszierenden, unreifen, pluripotenten Zellen.

19. Verfahren zur Detektion und Abtrennung von Zellen, die das Transkript nach einem der Ansprüche 1 bis 4 exprimieren, **dadurch gekennzeichnet, dass** es in situ die Durchführung einer RT-PCR umfasst, durch:
(a) Inkontaktbringen von Blutzellen mit einem markierten Primerpaar, wie es in einem der Ansprüche 13 bis 15 definiert ist, und
(b) Abtrennen der markierten Zellen durch jedes zweckdienliche Mittel.

20. Antikörper, **dadurch gekennzeichnet, dass** sie durch Immunisierung eines geeigneten Tiers mit einem Protein nach Anspruch 16 oder einem Peptid nach Anspruch 17 hergestellt werden und dass sie unter den HLA-G-Proteinen einzig das Protein nach Anspruch 16 oder das Peptid nach Anspruch 17 erkennen.

21. Verfahren zur Abtrennung von kernhaltigen fötalen Zellen, ausgehend von einer mütterlichen Blutprobe, **dadurch gekennzeichnet, dass** es umfasst:
(1) Inkontaktbringen der Probe aus mütterlichem Blut mit Antikörpern, die gegen die HLA-G-Proteine nach Anspruch 16 gerichtet sind, und
(2) Abtrennung der erhaltenen Komplexe fötale Zellen-Antikörper.

22. Verfahren zur spezifischen Abtrennung von zirkulierenden Lymphozyten, **dadurch gekennzeichnet, dass** es in situ die Durchführung einer RT-PCR umfasst, durch:
(a) Inkontaktbringen von Blutzellen mit einem markierten Primerpaar, wie es in einem der Ansprüche 13 bis 15 definiert ist, und
(b) zweckdienliche Abtrennung der markierten Zellen.

23. Medikament, **dadurch gekennzeichnet, dass** es ein Protein nach Anspruch 16 oder ein Peptid nach Anspruch 17 umfasst.

24. Immuntolerante Zusammensetzungen, **dadurch gekennzeichnet, dass** sie ein Protein nach Anspruch 16 oder ein Peptid nach Anspruch 17, kombiniert mit einem Vehikel oder einem Träger, das/der unter pharmazeutischen Gesichtspunkten zweckdienlich ist, umfassen.

## Claims

1. cDNA molecule, derived from an mRNA molecule of the human HLA-G gene of the major histocompatibility complex (MHC), **characterised in that** it consists, successively from 5' to 3', of the following fragments of the said HLA-G:
- the fragment encoding the signal peptide (exon 1),
- the fragment encoding the α₁ domain (exon 2),
- the fragment encoding the α₂ domain (exon 3),
- the fragment encoding the transmembrane domain TM (exon 5),
- the fragment encoding the cytoplasmic domain (exon 6), and
- the untranslated fragment 3' (exon 8), the said cDNA molecule being named HLA-G4.

2. Fragment of cDNA according to claim 1, **characterised in that** it consists, successively from 5' to 3', of:
- the fragment encoding the α₂ domain (exon 3),
- the fragment encoding the transmembrane domain TM (exon 5),
- the fragment encoding the cytoplasmic domain (exon 6), and
- the untranslated fragment 3' (exon 8).

3. Fragment according to claim 2, **characterised in that** it is defined by the sequence SEQ ID NO:1, encoding a protein in which the α₂ domain and the transmembrane sequence of HLA-G are directly joined.

4. Transcription product of the human HLA-G gene of CMH, isolated and purified, **characterised in that** it consists, successively from 5' to 3', of the following fragments of the HLA-G gene:
- the fragment encoding the signal peptide (exon 1),
- the fragment encoding the α₁ domain (exon 2),
- the fragment encoding the α₂ domain (exon 3),
- the fragment encoding the transmembrane domain TM (exon 5), and
- the fragment encoding the cytoplasmic domain (exon 6) of HLA-G.

5. Fragment of the cDNA molecule according to claim 1, **characterised in that** it is defined by the oligonucleotide of sequence SEQ ID NO:2.

6. Fragment of the cDNA molecule according to claim 1, **characterised in that** it is defined by the oligonucleotide of sequence SEQ ID NO:3.

7. Fragment of the cDNA molecule according to claim 1, **characterised in that** it is defined by the oligonucleotide of sequence SEQ ID NO:4.

8. Fragment of the cDNA molecule according to claim 1, **characterised in that** it is defined by the oligonucleotide of sequence SEQ ID NO:5.

9. Fragment of the cDNA molecule according to claim 1, **characterised in that** it is defined by the oligonucleotide of sequence SEQ ID NO:6.

10. Nucleotide probes, **characterised in that** they are selected from the group consisting of the molecules or fragments of nucleic acid according to any one of claims 1 to 9, marked by means of a marker such as a radioactive isotope, an appropriate enzyme or a fluorochrome.

11. Probe according to claim 10, **characterised in that** it consists of an oligonucleotide of sequence SEQ ID NO:6 according to claim 9.

12. Probe according to claim 10, **characterised in that** it consists of an oligonucleotide of sequence SEQ ID NO:4 according to claim 7.

13. Pairs of primers for amplifying a sequence according to any one of claims 1 to 4, **characterised in that** they are selected from the group consisting of the oligonucleotides of sequence SEQ ID NO:2 to 6.

14. Pairs of primers according to claim 13, **characterised in that** it consists of the oligonucleotide of sequence SEQ ID NO:2 according to claim 5, paired with the oligonucleotide of sequence SEQ ID NO:5 according to claim 8.

15. Pair of primers according to claim 13, **characterised in that** it consists of the oligonucleotide of sequence SEQ ID NO:3 according to claim 6, paired with the oligonucleotide of sequence SEQ ID NO:5 according to claim 8.

16. Protein or peptide fragment, **characterised in that** its sequence is that which is encoded by the molecule or fragment of nucleic acid according to any one of claims 1 to 4.

17. Peptide according to claim 16, **characterised in that** it is defined by the sequence SEQ ID NO:7.

18. Method of selection and enrichment with undifferentiated haematopoietic cells(immature blood cells or strain cells), **characterised in that** it comprises:
(a) the selection, according to circumstances, from amongst peripheral blood, umbilical cord blood or bone marrow, of a sample that has been taken,
(b) putting the said sample in contact with anti-CD34 antibodies.
(c) the separation of the complexes formed with cells containing a CD34 antigen and anti-CD34 antibodies.
(d) the implementation of a RT-PCR in situ on the cells obtained at step (c) in the presence of primers labelled by means of a fluorochrome, as defined in any one of claims 13 to 15,
(e) the separation of the fluorescent cells obtained, and
(f) the selection of the pluripotent immature non-fluorescent cells.

19. Method of detecting and separating cells expressing the transcript according to any one of claims 1 to 4, **characterised in that** it comprises, in situ, the implementation of a RT-PCR, by:
(a) putting blood cells in contact with a pair of labelled primers as defined in any one of claims 13 to 15, and
(b) separating the labelled cells by any suitable means.

20. Antibodies, **characterised in that** they are prepared by immunising an appropriate animal, with a protein according to claim 16 or a peptide according to claim 17, and **in that** they recognise, amongst the HLA-G proteins, only the said protein according to claim 16 or the said peptide according to claim 17.

21. Method of separating nucleated foetal cells, from a sample of maternal blood, **characterised in that** it comprises:
(1) putting the sample of maternal blood in contact with antibodies directed against the HLA-G proteins according to claim 16, and
(2) separating the foetal cell/antibody complexes obtained.

22. Method for the specific separation of circulating lymphocytes, **characterised in that** it comprises, in situ, the implementation of a RT-PCR, by:
(a) putting blood cells in contact with a pair of labelled primers as defined in any one of claims 13 to 15, and
(b) suitably separating the labelled cells.

23. Medicament, **characterised in that** it comprises a protein according to claim 16 or a peptide according to claim 17.

24. Immunotolerant compositions, **characterised in that** they comprise a protein according to claim 16 or a peptide according to claim 17, associated with a pharmaceutically acceptable vehicle or support.


### LISTE DE SEQUENCES

(1) INFORMATIONS GENERALES:
   (i) DEPOSANT:
      (A) NOM: COMMISSARIAT A L'ENERGIE ATOMIQUE (CEA)
      (B) RUE: 31-33 RUE DE LA FEDERATION
      (C) VILLE: PARIS
      (E) PAYS: FRANCE
      (F) CODE POSTAL: 75015
   (ii) TITRE DE L'INVENTION: TRANSCRIS DU GENE DE CMH DE CLASSE I HLA-G ET LEURS APPLICATIONS
   (iii) NOMBRE DE SEQUENCES: 7
   (iv) FORME DECHIFFRABLE PAR ORDINATEUR:
      (A) TYPE DE SUPPORT: Floppy disk
      (B) ORDINATEUR: IBM PC compatible
      (C) SYSTEME D'EXPLOITATION: PC-DOS/MS-DOS
      (D) LOGICIEL: PatentIn Release #1.0, Version #1.30 (OEB)
   (vi) DONNEES DE LA DEMANDE ANTERIEURE:
      (A) NUMERO DE LA DEMANDE: FR 9403179
      (B) DATE DE DEPOT: 18-MAR-1994
(2) INFORMATIONS POUR LA SEQ ID NO: 1:
   (i) CARACTERISTIQUES DE LA SEQUENCE:
      (A) LONGUEUR: 443 paires de bases
      (B) TYPE: nucléotide
      (C) NOMBRE DE BRINS: simple
      (D) CONFIGURATION: linéaire
   (ii) TYPE DE MOLECULE: ADNc pour ARNm
   (xi) DESCRIPTION DE LA SEQUENCE: SEQ ID NO: 1:
   CCAATGTGGC TGAACAAAGG AGAGCCTACC TGGAGGGCAC GTGCGTGGAG TGGCTCCACA   60
   GATACCTGGA GAACGGGAAG GAGATGCTGC AGCGCGCGGA GCAGTCTTCC CTGCCCACCA  120
   TCCCCATCAT GGGTATCGTT GCTGGCCTGG TTGTCCTTGC AGCTGTAGTC ACTGGAGCTG  180
   CGGTCGCTGC TGTGCTGTGG AGRAAGAAGA GCTCAGATTG AAAAGGAGGG AGCTACTCTC  240
   AGGCTGCAAT GTGAAACAGC TGCCCTGTGT GGGACTGAGT GGCAAGTCCC TTTGTGACTT  300
   CAAGAACCCT GACTTCTCTT TSTGCAGAGA CCAGCCCACC CCTGTGCCCA CCATGACCCT  360
   CTTYCTCATG CTGAACTGCA TTCCTTCCCC AATCACCTTT CCTGTTCCAG AAAAGGGGCT  420
   GGGATGTCTC CGTCTCTGTC TCA                                          443
(2) INFORMATIONS POUR LA SEQ ID NO:2:
   (i) CARACTERISTIQUES DE LA SEQUENCE:
      (A) LONGUEUR: 21 paires de bases
      (B) TYPE: nucléotide
      (C) NOMBRE DE BRINS: simple
      (D) CONFIGURATION: linéaire
   (ii) TYPE DE MOLECULE: ADNc pour ARNm
   (xi) DESCRIPTION DE LA SEQUENCE: SEQ ID NO: 2:
   GGAAGAGGAG ACACCGGAAC A   21
(2) INFORMATIONS POUR LA SEQ ID NO:3:
   (i) CARACTERISTIQUES DE LA SEQUENCE:
      (A) LONGUEUR: 20 paires de bases
      (B) TYPE: nucléotide
      (C) NOMBRE DE BRINS: simple
      (D) CONFIGURATION: linéaire
   (ii) TYPE DE MOLECULE: ADNc pour ARNm
   (xi) DESCRIPTION DE LA SEQUENCE: SEQ ID NO: 3:
   CCAATGTGGC TGAACAAAGG 20
(2) INFORMATIONS POUR LA SEQ ID NO: 4:
   (i) CARACTERISTIQUES DE LA SEQUENCE:
      (A) LONGUEUR: 20 paires de bases
      (B) TYPE: nucléotide
      (C) NOMBRE DE BRINS: simple
      (D) CONFIGURATION: linéaire
   (ii) TYPE DE MOLECULE: ADNc pour ARNm
   (xi) DESCRIPTION DE LA SEQUENCE: SEQ ID NO: 4:
   CCCCTTTTCT GGAACAGGAA 20
(2) INFORMATIONS POUR LA SEQ ID NO: 5:
   (i) CARACTERISTIQUES DE LA SEQUENCE:
      (A) LONGUEUR: 20 paires de bases
      (B) TYPE: nucléotide
      (C) NOMBRE DE BRINS: simple
      (D) CONFIGURATION: linéaire
   (ii) TYPE DE MOLECULE: ADNc pour ARNm
   (xi) DESCRIPTION DE LA SEQUENCE: SEQ ID NO: 5:
   TGAGACAGAG ACGGAGACAT 20
(2) INFORMATIONS POUR LA SEQ ID NO:6:
   (i) CARACTERISTIQUES DE LA SEQUENCE:
      (A) LONGUEUR: 20 paires de bases
      (B) TYPE: nucléotide
      (C) NOMBRE DE BRINS: simple
      (D) CONFIGURATION: linéaire
   (ii) TYPE DE MOLECULE: ADNc pour ARNm
   (xi) DESCRIPTION DE LA SEQUENCE: SEQ ID NO: 6:
   CAGCGCGCGG AGCAGTCTTC 20
(2) INFORMATIONS POUR LA SEQ ID NO: 7:
   (i) CARACTERISTIQUES DE LA SEQUENCE:
      (A) LONGUEUR: 72 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 7:
   Asn Val Ala Glu Gln Arg Arg Ala Tyr Leu Glu Gly Thr Cys Val Glu
   1               5                   10                  15
   Trp Leu His Arg Tyr Leu Glu Asn Gly Lys Glu Met Leu Gln Arg Ala
               20                  25                  30
   Glu Gln Ser Ser Leu Pro Thr Ile Pro Ile Met Gly Ile Val Ala Gly
           35                  40                  45
   Leu Val Val Leu Ala Ala Val Val Thr Glu Ala Ala Val Ala Ala Val
       50                  55                  60
   Leu Trp Arg Lys Lys Ser Ser Asp
   65                  70